# DEMANDE DE BREVET EUROPEEN

(11) **EP 0 974 433 A1**
(43) Date de publication de la demande: **26.01.2000**
(21) Numéro de dépôt: 99490020.7
(22) Date de dépôt: 16.07.1999
(51) Int. Cl.: B26F 1/00, B26F 1/26, A61F 13/15, D04H 13/00, B32B 31/00, B29C 59/06

(54) **Procédé et dispositif de perforation d'une nappe non tissée de fibres ou filaments**

(30) Priorité: 23.07.1998 FR 9809579
(71) Demandeur: Albis, 13060 Roasio - Curavecchio (IT)
(72) Inventeur: Boscolo, Galliano G., 13060 Vigliano (BI) (IT)
(74) Mandataire: Hénnion, Jean-Claude

(57) **Abrégé**

Le procédé de l'invention consiste à :
- amener une nappe non-tissée (19) de fibres ou filaments en contact avec la première face d'une pièce (6) de faible épaisseur pourvue d'au moins un orifice débouchant (7),
- déplacer, notamment par aspiration, vers l'intérieur de l'orifice (7) les fibres ou filaments, au droit dudit orifice (7), en sorte que certaines fibres ou filaments soient en élévation par rapport à l'autre face (6a) de la pièce (6),
- et couper lesdites fibres ou fils en élévation.

Le dispositif comprend :
- une pièce (6) de faible épaisseur pourvue d'au moins un orifice débouchant (7),
- un moyen apte à assurer le déplacement des fibres ou filaments vers l'intérieur de l'orifice (7), au-delà de l'autre face (6a) de la pièce (6)
   et un moyen de coupe (16a) des fibres ou filaments dépassant de ladite autre face (6a).

## Description

La présente invention concerne le domaine des nappes, non tissées, constituées de fibres ou de filaments, destinées notamment mais non exclusivement à être utilisées pour la réalisation d'articles d'hygiène, par exemple pour des couches-culottes pour bébés ou pour incontinents adultes ou encore des protections féminines. Elles concernent plus particulièrement un procédé et un dispositif aptes à produire des perforations dans une telle nappe non tissée.

Le fait de former des perforations dans un matériau du type nappe non tissée voire film plastique est déjà connu pour favoriser par diffusion capillaire le passage de fluide à travers ledit matériau.

Plus particulièrement le document EP 0214608 décrit un dispositif et un procédé pour réaliser des perforations à travers un non tissé qui, de manière spécifique, est composé essentiellement de fibres thermoplastiques. Le dispositif en question comprend deux organes entre lesquels passe la nappe de fibres. Le premier organe supporte une pluralité de plots tandis que l'autre organe comprend des évidements disposés de telle sorte que les extrémités des plots pénètrent dans la nappe et repoussent certaines fibres. Les plots et le contour des évidements du second organe sont chauffés en sorte que les fibres thermoplastiques qui ont été ainsi écartées soient liées les unes aux autres. On réalise ainsi des perforations dont les contours périphériques sont consolidés, étant formés de fibres liées les unes aux autres.

Selon le demandeur, le procédé mis en oeuvre dans le document EP 0214608 présente comme inconvénients d'une part de nécessiter l'utilisation de fibres thermoplastiques et d'autre part d'apporter, au niveau des perforations, une certaine rigidité due à la présence, au niveau des perforations, des fibres soudées les unes aux autres.

Le but que s'est fixé le demandeur est de proposer un procédé de perforation d'une nappe non tissée de fibres ou de filaments qui pallie les inconvénients précités.

De manière caractéristique, selon l'invention, le procédé consiste à :
- amener la nappe non tissée en contact avec une première face d'une pièce de faible épaisseur pourvue d'au moins un orifice débouchant,
- déplacer vers l'intérieur de l'orifice les fibres ou filaments, constitutifs de la nappe, au droit dudit orifice, de sorte que certaines fibres ou filaments soient en élévation par rapport à l'autre face de la pièce,
- et couper lesdites fibres ou filaments en élévation.

Ainsi, selon le principe général de la présente invention, chaque perforation est réalisée, dans un premier temps, en déformant localement la nappe en sorte de déplacer, dans une direction sensiblement perpendiculaire au plan général de ladite nappe, certaines fibres, et, dans un second temps, à sectionner les fibres ou filaments au niveau de cette déformation.

Dans une variante préférée de réalisation, le déplacement des fibres ou filaments, vers l'intérieur de l'orifice, est réalisé par aspiration.

Avantageusement, la coupe des fibres ou filaments en élévation par rapport à l'autre face de la pièce se fait par déplacement d'un couteau selon cette autre face , alors que les fibres ou filaments sont bloqués en position au niveau de l'orifice. S'il n'y avait pas de blocage en position des fibres ou filaments, au niveau de l'orifice, il est vraisemblable qu'au moins une partie desdites fibres ou filaments serait repoussée vers l'intérieur de l'orifice lors du passage du couteau.

C'est un autre objet de l'invention que de proposer une installation spécialement conçue pour réaliser la perforation d'une nappe non tissée de fibres ou de filaments, selon le procédé précité.

De manière connue par le document EP0214608, cette installation comprend une pièce pourvue d'au moins un orifice débouchant et un moyen de déplacement des fibres ou filaments à l'intérieur de l'orifice. Dans le document antérieur, ce moyen de déplacement est constitué par le plot chauffant.

De manière caractéristique, selon l'invention, la pièce est de faible épaisseur et le moyen de déplacement est apte à assurer le déplacement des fibres ou filaments à travers l'orifice au-delà de l'autre face de la pièce ; de plus l'installation comprend un moyen de coupe des fibres ou filaments dépassant de ladite autre face.

Dans une variante préférée de réalisation, la pièce de faible épaisseur est un cylindre perforé tournant et le moyen de déplacement des fibres ou filaments est un système d'aspiration intérieur audit cylindre.

Dans ces conditions, la nappe non tissée de fibres ou filaments est appliquée sur la première face, extérieure, du cylindre tournant et, au niveau du système d'aspiration, les fibres ou filaments pénètrent, sous l'effet de l'aspiration, à l'intérieur des orifices formés dans le cylindre jusqu'à dépasser au-delà de l'autre face, intérieure, dudit cylindre.

De préférence le moyen de coupe est un couteau fixe disposé à l'intérieur du cylindre et dont la lame de coupe vient au niveau de l'autre face intérieure du cylindre.

Pour que la coupe des fibres ou filaments se fasse de manière nette sur toute la zone en élévation par rapport à la face intérieure du cylindre, il est nécessaire que lesdites fibres ou filaments soient bloqués en position au niveau de chaque orifice du cylindre perforé.

Pour obtenir ce blocage en position, de préférence l'installation selon l'invention comporte au moins un plot de blocage synchronisé avec les moyens de déplacement pour s'appliquer sur la périphérie extérieure de l'orifice, après le déplacement des fibres ou filaments vers l'intérieur de l'orifice. Le plot ne pénètre pas à proprement parler à l'intérieur de l'orifice mais a une configuration qui est telle qu'il vient coincer les fibres qui délimitent, au niveau de la surface du cylindre perforé, la base de la zone déformée. De préférence chaque plot de blocage est supporté par un cylindre dont la rotation est synchronisée avec la rotation du cylindre perforé aspirant. Cette disposition particulière a pour effet, lors de l'application du plot de blocage sur les fibres, d'entraîner un léger déplacement desdites fibres ce qui favorise l'action de la lame de coupe.

La nappe non tissée perforée obtenue grâce au procédé ou à l'aide du dispositif de l'invention peut être utilisée dans tous les domaines où la présence de perforations est recherchée, et en particulier dans le domaine des articles d'hygiène, notamment en contact avec la peau de l'utilisateur, comme couverture du matelas absorbant.

Elle peut être utilisée seule ou en combinaison avec d'autres nappes. Dans ce dernier cas, il s'agira plus particulièrement d'un non tissé composite, constitué de la superposition et de l'assemblage, par exemple par thermoliage, de plusieurs nappes parmi lesquelles au moins une sera une nappe non tissée perforée conformément à l'invention. Bien sûr le choix des fibres ou filaments constitutifs de chaque nappe sera déterminé en fonction de l'application recherchée.

Par exemple un non tissé composite peut être constitué de deux voiles de carde, dont l'un est perforé conformément à l'invention, les deux voiles étant ensuite assemblés par thermoliage.

Par exemple un non tissé composite peut être constitué par l'assemblage de deux voiles de carde entre lesquels est disposée une nappe de filaments continus perforée, du type Meltblown.

De plus, que la nappe non tissée perforée soit utilisée seule ou en tant que constituant d'un non tissé composite, elle peut, en fonction des applications, n'être perforée que sur une portion de sa surface limitée à ce qui sera, lors de la fabrication de l'article définitif, une zone d'acquisition de liquide.

La présente invention sera mieux comprise à la lecture de la description qui va être faite d'un dispositif de perforation d'une nappe non tissée et d'exemples d'utilisation d'une telle nappe, illustrée par le dessin annexé dans lequel :
La figure 1 est une représentation schématique partielle en coupe du dispositif de perforation constitué d'un premier cylindre tournant aspirant et d'un second cylindre porteur de plots de blocage,
La figure 2 est une vue partielle agrandie du dispositif de la figure 1, dans la zone où un plot de blocage s'applique contre un orifice du premier cylindre perforé,
La figure 3 est une représentation très schématisée d'une ligne complète de fabrication d'un non tissé tri-couches perforé.

La présente invention a pour objet la réalisation de perforations dans une nappe non tissée de fibres ou de filaments.

Le dispositif 1 spécialement conçu pour la réalisation de telles perforations est constitué d'un premier cylindre 2 qui est un cylindre perforé aspirant, entraîné en rotation autour de son axe longitudinal 3, et d'un second cylindre 4, entraîné en rotation autour de son axe longitudinal 5, parallèle à l'axe 3 du premier cylindre 2.

Le premier cylindre 2 comporte, sur sa périphérie extérieure, une pièce cylindrique, de faible épaisseur, munie d'orifices débouchants 7. Cette pièce extérieure 6 est entraînée en rotation dans le sens de la flèche F. Ce premier cylindre 2 comporte également une pièce intérieure cylindrique 8, de même axe longitudinal 3. Cette pièce intérieure 8 est fixe et délimite une chambre interne 9 à laquelle est raccordée une source d'aspiration non représentée.

Entre la pièce extérieure 6 et la pièce intérieure 8 est constituée une chambre d'aspiration 10 qui met en relation la chambre interne 9 et une portion angulaire déterminée 11 de la pièce extérieure 6. Cette chambre d'aspiration 10 est délimitée d'une part par deux montants 12, 13 fixés sur la pièce intérieure 8 de part et d'autre d'une ouverture 14 pratiquée dans ladite pièce 8 et d'autre part par une lèvre d'étanchéité 15 et une lame 16 s'étendant respectivement entre chaque montant 12, 13 et la face intérieure 6a de la pièce extérieure 6. La lèvre d'étanchéité 15 peut notamment être constituée d'une bande en caoutchouc dont l'extrémité 15a est en contact avec ladite face intérieure 6a. La lame 16 est terminée par une extrémité coupante formant une lame de coupe 16a, inclinée vers l'intérieur de la chambre d'aspiration 10. Dans le sens de rotation F du premier cylindre 2, la lèvre d'étanchéité 15 est disposée la plus en amont et la lame 16 la plus en aval.

Lorsque la source d'aspiration est en fonctionnement, il se crée une dépression dans la chambre interne 9 qui provoque un courant d'aspiration à travers la chambre d'aspiration 10, y compris à travers les orifices 7 de la pièce extérieure 6 se trouvant au niveau de la portion angulaire 11 comprise entre les extrémités 15a et 16a de la lèvre d'étanchéité 15 et de la lame 16.

Le second cylindre 4 est muni, sur sa périphérie, de plots de blocage 17 qui sont agencés pour coopérer avec les orifices 7 pratiqués dans la pièce extérieure 6 du premier cylindre 2.

Plus précisément chaque plot de blocage 17 se présente sous la forme d'un élément cylindrique terminé par un bout hémisphérique 18 qui est d'un diamètre légèrement supérieur au diamètre de l'orifice 7, avec lequel il doit coopérer.

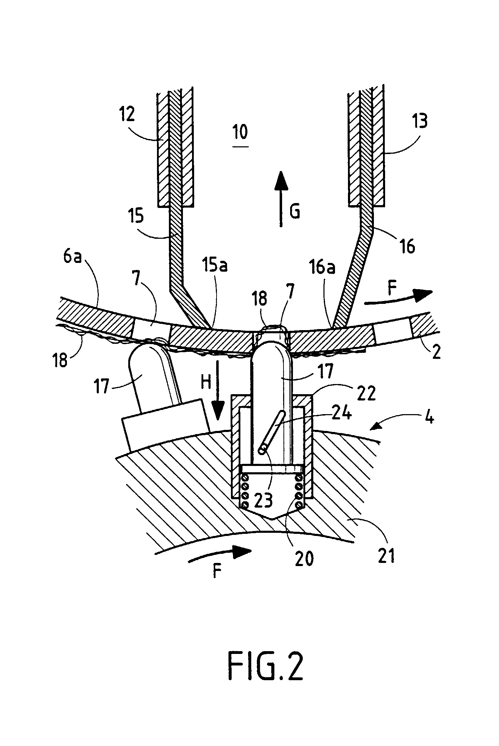

Les deux cylindres 2, 4 sont synchronisés en sorte que lors de leur rotation respective selon les flèches F, le bout hémisphérique 18 d'un plot 17 donné vient s'appliquer précisément sur le contour périphérique 7a de l'orifice 7 correspondant, comme cela apparaît clairement à l'examen de la figure 2.

La réalisation de perforations à l'aide du dispositif 1 s'effectue dans les conditions suivantes.

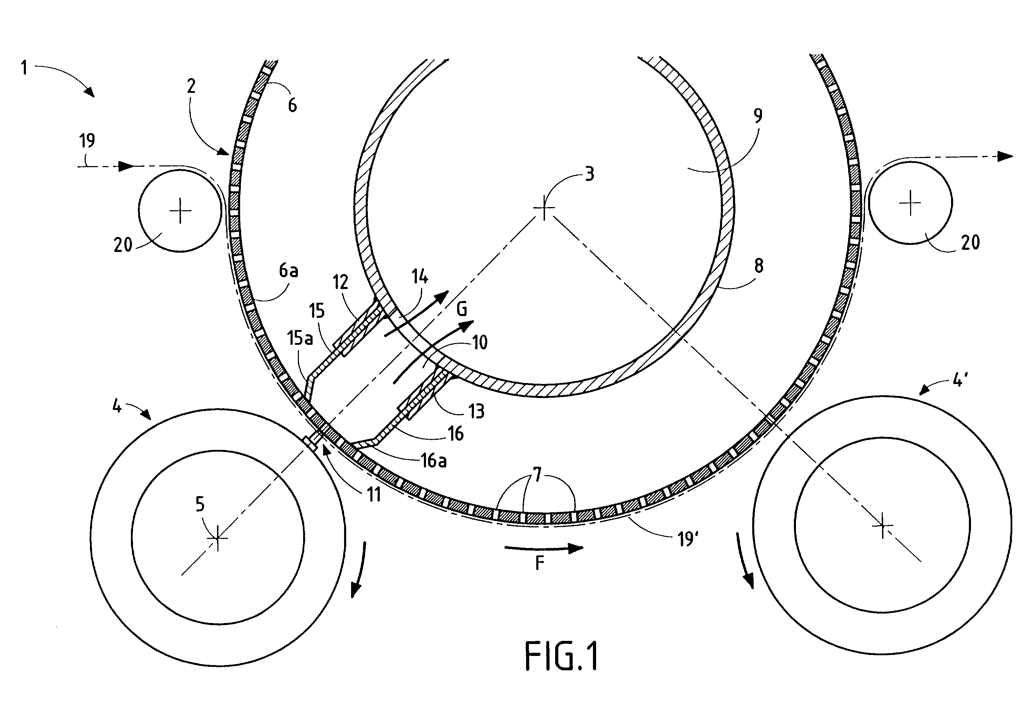

La nappe non tissée 19, constituée de fibres ou de filaments, provenant par exemple d'une carde est appliquée sur le premier cylindre perforé 2 sur une portion de celui-ci délimitée par deux rouleaux applicateurs 20, entraînés en rotation à la même vitesse linéaire que le premier cylindre perforé 2. Lors de son déplacement sur ledit cylindre perforé 2, la nappe de fibres 19 passe au niveau de la portion angulaire 11 qui est en relation avec la chambre d'aspiration 10. Du fait du courant d'aspiration intervenant à travers les orifices 7 localisés dans cette portion angulaire 11, les fibres ou filaments qui se trouvent au droit de chaque orifice 7 ont tendance à se déplacer vers l'intérieur de l'orifice 7 de sorte que certaines fibres ou filaments sont en élévation par rapport à la face intérieure 6a de la pièce extérieure 6 du premier cylindre perforé 2. Ce déplacement des fibres ou filaments intervient dès que l'orifice 7, au droit desdites fibres ou filaments, est passé au-delà de l'extrémité 15a de la lèvre d'étanchéité 15. Cette déformation des fibres ou filaments est ensuite bloquée en position par application du bout hémisphérique 18 du plot de blocage 17 venant coopérer avec l'orifice 7 correspondant. Les portions de fibres ou filaments, en élévation par rapport à la face intérieure 6a de la pièce 6 sont enfin coupés par la lame de coupe 16a de la lame 16, ce qui réalise la perforation recherchée. Les fibres ou filaments découpés sont emmenés dans le courant d'aspiration G et évacués. La nappe non tissée perforée 19' continue son déplacement sur le premier cylindre perforé 2. Eventuellement, comme illustré à la figure 1, elle peut passer devant un autre second cylindre 4' muni de plots de blocage s'il est souhaitable de pratiquer à d'autres endroits ou selon d'autres tailles, d'autres perforations sur la nappe 19'.

Concernant les plots de blocage 17, le bout hémisphérique 18 a une configuration telle que, lorsqu'il est en appui sur le contour périphérique 7a de l'orifice 7, il ne déborde pas au-delà de la face intérieure 6a de la pièce 6. De plus, de préférence il est monté sur ressort 20 de manière à compenser les écarts de réglage éventuels et éviter un écrasement intempestif des fibres ou filaments. La fonction du plot de blocage 17 est de maintenir en position les fibres ou filaments 21 déplacés vers l'intérieur de l'orifice 7 de telle sorte que la lame de coupe 16a puisse couper précisément les parties de fibres ou filaments débordant, dans la chambre d'aspiration 10, au-delà de la face intérieure 6a de la pièce 6. Il est également à noter que le bout hémisphérique 18 du plot 17 vient obturer progressivement l'orifice 7, au fur et à mesure de la rotation des deux cylindres 2, 4 de sorte que lors du blocage définitif en position des fibres ou filaments et lors de la coupe, l'orifice 7 est totalement obturé. Cette obturation progressive permet également de limiter le déplacement des fibres ou filaments vers l'intérieur de la chambre d'aspiration 10 du fait qu'il existe déjà un pincement partiel de certaines fibres ou filaments par le plot de blocage 17 avant même le passage de l'orifice 7 dans la chambre d'aspiration 10.

Dans une variante de réalisation, le plot 17 de blocage est monté dans la pièce extérieure 21 du second cylindre 4 par l'intermédiaire d'un logement 22 au fond duquel est disposé le ressort 20. Le plot 17 est agencé à l'intérieur du logement 22 en sorte qu'il tourne sur lui-même lorsqu'il se déplace dans le sens de la flèche H lors de la compression du ressort 20. Plus précisément le plot 17 est muni d'une goupille 23, en saillie à l'intérieur du logement 22, cette goupille pénétrant dans une coulisse 23 formée dans la paroi dudit logement 22 et qui est inclinée par rapport à la direction H de déplacement en hauteur du plot 17. Ainsi lorsque le bout hémisphérique 18 du plot 17 vient s'appliquer sur la pièce extérieure 6 lors de la rotation des deux cylindres 2, 4, cet embout hémisphérique 18 est repoussé dans la direction H du fait de la présence des fibres ou filaments constitutifs de la nappe 19. Ce déplacement peut être plus ou moins important en fonction de l'épaisseur de ladite nappe.

Lors de ce déplacement en hauteur du plot 17, la goupille 23 se déplace corrélativement dans la coulisse 24. Du fait de l'inclinaison de cette coulisse 24, le plot 17 effectue une légère rotation sur lui-même lors de son déplacement dans la direction H. Cette légère rotation a pour effet de déplacer également les fibres ou filaments les uns par rapport aux autres et d'obtenir une meilleure présentation desdites fibres ou filaments lors de leur passage au niveau de la lame de coupe 16a.

Dans l'exemple qui vient d'être décrit, la nappe non tissée 19 est perforée de manière continue dans la zone où le second cylindre 4 vient coopérer avec les orifices 7 du premier cylindre 2. On comprend que cette zone peut faire toute la largeur de la nappe ou peut ne faire qu'une partie de cette largeur selon l'application qui est visée. En particulier lorsque cette nappe 19 est mise en oeuvre pour la réalisation d'un article d'hygiène pour favoriser le passage de liquide, il peut être judicieux de ne perforer ladite nappe 19 que dans la zone qui, dans l'article confectionné, correspondra à l'acquisition de liquide.

De même il est possible de ne pas réaliser de perforations en continu sur toute la longueur de la nappe 19 mais de fractionner cette longueur en portions perforées et en portions non perforées, par exemple en régulant en conséquence l'aspiration dans la chambre interne 9 ou en éloignant légèrement l'un de l'autre les axes 3, 5 de rotation des deux cylindres 2, 4.

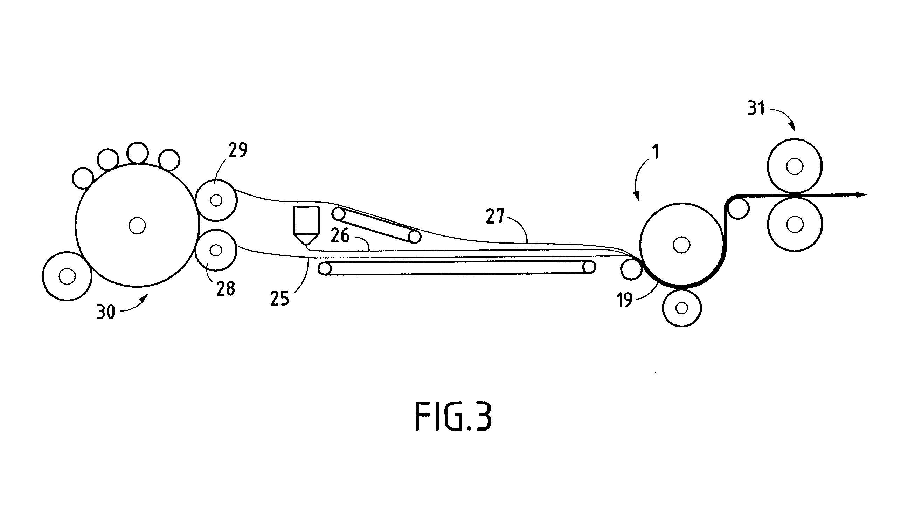

Dans l'exemple de réalisation qui est illustré à la figure 3, la nappe non tissée 19 qui est perforée grâce au dispositif 1 est une nappe composée de la superposition de trois couches 25, 26, 27, les couches externes 25, 27 étant deux voiles de carde provenant des deux peigneurs 28, 29 d'une carde 30. La couche 26 intermédiaire est constituée de filaments extrudés soufflés du type Meltblown. Ainsi on obtient une nappe composite 19' constituée des trois dites couches 25, 26, 27, dont les perforations se font par la coupe des éléments fibreux ou filamentaires provenant des trois couches successives.

On comprend qu'il est possible de faire varier la disposition respective des organes de l'installation illustrée à la figure 3 pour réaliser des perforations uniquement sur l'une et/ou l'autre des trois couches précitées. Par exemple il serait envisageable de détourner le parcours des voiles de carde constituant les couches extérieures 25, 27 de manière à ce que seule la couche intermédiaire 26 du type Meltblown soit perforée. Dans un exemple précis de réalisation, on a obtenu un non tissé composite à trois couches 25, 26, 27 constituées respectivement de deux voiles de carde de 10 g/m² pour les couches externes 25, 27 et une nappe Meltblown de 3g/m² pour la couche intermédiaire 26.

Dans l'exemple illustré à la figure 3, il est question de la superposition de trois couches. Bien sûr il est possible de superposer uniquement deux couches voire plus de trois couches.

De plus le caractère hydrophile ou hydrophobe des fibres ou filaments constitutifs de chaque couche, voire de la nappe elle-même, est un des paramètres sur lesquels il est possible d'intervenir en fonction des applications recherchées.

Il est à souligner que le déplacement des fibres ou filaments, lors de l'aspiration, est d'autant plus facile que ces fibres ou filaments ont un degré important de liberté dans la nappe de départ (en une ou plusieurs couches). Il est donc préférable que les moyens mécaniques ou thermiques d'entremêlement ou de liaison des fibres ou filaments interviennent après qu'aient été réalisées les perforations conformément à l'invention. Dans l'installation illustrée à la figure 3, la nappe 19' perforée passe ensuite dans un ensemble 31 de thermoliage.

## Revendications

1. Procédé de perforation d'une nappe non-tissée (19) de fibres ou filaments caractérisé en ce qu'il consiste à :
- amener la nappe (19) en contact avec une première face d'une pièce (6) de faible épaisseur pourvue d'au moins un orifice débouchant (7),
- déplacer vers l'intérieur de l'orifice (7) les fibres ou filaments, au droit dudit orifice (7), en sorte que certaines fibres ou filaments soient en élévation par rapport à l'autre face (6a) de la pièce (6),
- et couper lesdites fibres ou filaments en élévation.

2. Procédé selon la revendication 1 caractérisé en ce que le déplacement est réalisé par aspiration.

3. Procédé selon l'une des revendications 1 ou 2 caractérisé en ce que la coupe se fait par déplacement d'un couteau (16a) au droit de ladite autre face (6a) de la pièce (6) alors que les fibres ou filaments sont bloqués en position au niveau de l'orifice (7).

4. Dispositif de perforation d'une nappe non-tissée (19) de fibres ou filaments comprenant :
- une pièce (6) pourvue d'au moins un orifice débouchant (7),
- un moyen de déplacement des fibres ou filaments vers l'intérieur de l'orifice (7),
caractérisé en ce que :
- la pièce (6) est de faible épaisseur,
- le moyen de déplacement est apte à assurer le déplacement des fibres ou filaments à travers l'orifice (7)
et en ce qu'elle comprend un moyen de coupe (16a) des fibres ou filaments dépassant de l'orifice (7).

5. Dispositif selon la revendication 4 caractérisée en ce que la pièce (6) est un cylindre perforé tournant (2) et le moyen de déplacement est un système d'aspiration intérieur audit cylindre.

6. Dispositif selon la revendication 5 caractérisée en ce que le moyen de coupe est un couteau fixe disposé à l'intérieur du cylindre (2) et dont la lame de coupe (16a) vient au niveau de la face intérieure (6a) du cylindre (6).

7. Dispositif selon la revendication 6 caractérisé en ce qu'il comporte au moins un plot de blocage (17) synchronisé avec les moyens de déplacement pour s'appliquer sur la périphérie extérieure (7a) de l'orifice (7), après le déplacement des fibres ou filaments vers l'intérieur de l'orifice (7).

8. Dispositif selon la revendication 7 caractérisé en ce que le ou les plots (7) sont supportés par un second cylindre (4) dont la rotation est synchronisée avec la rotation du cylindre perforé aspirant (2).

9. Dispositif selon la revendication 8 caractérisé en ce que l'orifice (7) est circulaire et a un diamètre donné, en ce que chaque plot (17) correspondant est terminé par un bout semi-sphérique (18) de diamètre supérieur au diamètre donné, et en ce que chaque plot (17) est monté sur un ressort de pression (20).

10. Dispositif selon la revendication 9 caractérisé par un moyen de rotation du plot (17) sur lui-même lors de son déplacement en hauteur (H) sous l'effet du ressort (20).

11. Dispositif selon la revendication 10 caractérisé en ce que chaque plot (17) est muni d'une goupille (23) en saillie et est dans un logement (22) pourvu d'une coulisse (24) inclinée par rapport à la direction de déplacement en hauteur du plot (17) et dans laquelle se déplace la goupille (23).

12. Non-tissé composite perforé constitué de la superposition et du liage d'au moins deux nappes non-tissées de fibres ou filaments, parmi lesquelles au moins une nappe perforée selon le procédé de l'une des revendications 1 à 3 ou avec le dispositif selon l'une des revendications 4 à 11.

13. Non-tissé composite perforé selon la revendication 12 caractérisé en ce qu'il est constitué de la superposition et du liage de deux nappes externes chacune étant formée d'un voile de carde et d'une nappe intermédiaire, en filament extrudé/soufflé (Meltblown).
